# FASCICULE DE BREVET EUROPEEN

(11) **EP 3 633 304 B1**
(45) Date de publication et mention de la délivrance du brevet: **09.12.2020**
(21) Numéro de dépôt: 19199574.5
(22) Date de dépôt: 25.09.2019
(51) Int. Cl.: F28D 20/02, A61N 1/39, F25D 3/00, F25D 11/00, B65D 81/18, B65D 85/50, A61J 1/16, A61B 50/00, H01M 10/615, H01M 10/62

(54) **BOÎTIER THERMO- REGULÉ AUTONOME**
AUTONOMES WÄRMEREGULIERTES GEHÄUSE
STANDALONE REGULATED THERMO-BOX

(30) Priorité: 04.10.2018 FR 1801050
(43) Date de publication de la demande: 08.04.2020
(73) Titulaire: Pyrescom, 66680 Canohes (FR); Université de Perpignan Via Domitia, 66100 Perpignan (FR)
(72) Inventeur: RIFFARD, Olivier, 66000 PERPIGNAN (FR); PY, Xavier, 66200 LATOUR-BAS-ELNE (FR)
(74) Mandataire: Rataboul, Xavier

(56) Documents cités:
- US-A- 4 928 501
- US-A1- 2016 243 000
- US-A1- 2018 133 495

## Description

La présente invention concerne un boîtier (ou armoire) thermo-régulé, autonome énergétiquement, pour conserver un produit thermo sensible à stocker, tel qu'un matériel médical (notamment un défibrillateur automatisé externe, ou « DAE »), des produits médicaux (médicaments et vaccins), des produits alimentaires, des liquides, des produits chimiques, des végétaux et matières organiques, des gaz conditionnés. Un tel boîtier est connu de US 4 928 501 A, qui forme la préambule de la revendication 1, US 2016/243000 A1 ou US 2018/133495 A1.

Le boitier selon la présente invention est destiné à être installé en extérieur, en poste fixe ou transportable, et fonctionne sans aucune connexion physique (électrique et réseau). Il maintient lesdits produits dans une plage de températures (température minimale, température maximale) acceptable pour leur utilisation finale.

Plus particulièrement, la présente invention fonctionne en refroidissement et/ou réchauffement par la combinaison d'effets physiques et mécaniques conjugués basés sur des énergies renouvelables et ce, dans les différentes zones habitées de la planète.

Le boîtier (par exemple une armoire) selon l'invention est un espace de stockage mobile ou statique, conçu pour être installé dans des zones non raccordées au réseau électrique (par exemple des plages, terrains de sport, chemins de randonnées) pour mettre à disposition le produit thermosensible, par exemple, un défibrillateur, contenu dans le boîtier.

De nombreux produits sont soumis à des contraintes thermiques contraires à leurs plages de températures acceptables lors de leurs transports ou lors de leurs stockages en extérieurs du fait des variations de températures entre le jour et la nuit et des variations climatiques dépendantes des saisons.

À ce jour les containers permettant le transport ou le stockage en extérieur de ces produits doivent disposer de dispositifs de climatisation alimentés par des sources d'énergies électriques ou thermiques non autonomes (groupe électrogène ou réseau électrique).

Ils ne peuvent donc être installés que dans des zones facilement accessibles ou reliées au réseau électrique.

Il a déjà été proposé des systèmes de stockage autonome, comme par exemple les boîtiers de la gamme ALCATHERM de la société COLDWAY.

Ces boîtiers utilisent un système thermochimique dont l'autonomie est limitée et qui doit être rechargé à partir d'une source d'énergie.

L'inconvénient de l'état de l'art actuel est qu'il impose le raccordement des boîtiers aux sources d'énergies précitées ce qui parfois n'est pas réalisable ou qui dans le cas de l'utilisation d'énergies thermiques implique l'utilisation d'énergie fossiles.

Les réalisations antérieures ne sont autonomes que sur une durée de temps limitée tout au plus à quelques heures et impliquent une recharge sur une source de courant au-delà de ce délai.

De plus, elles sont onéreuses car elles imposent des solutions complexes (électrovannes et circuit hermétique fermé) et des composants sensibles gaz NH3 (ammoniac), ce qui peut les rendre dangereuses en cas détérioration ou de dégradation volontaire entrainant une rupture du circuit.

La présente invention vise donc à proposer un boîtier simple, autonome et économe en énergie, tout en étant capable de stocker un produit thermosensible dans sa gamme de température de stockage optimale, et pouvant être installé de manière très polyvalente dans des zones non raccordées au réseau électrique.

Plus particulièrement, l'invention vise à permettre le stockage d'un produit thermosensible dans des températures prédéfinies, avec de faibles écarts de variations sur des plages de 24 heures, en utilisant les contraintes en températures (variations) rencontrées entre le jour et la nuit mais aussi été comme hiver (lorsque le contenu est limité en température respectivement haute et basse).

L'invention propose de combiner un volume de matériau à changement de phase avec un moyen de chauffage et un circuit de circulation d'air entre le matériau à changement de phase et le produit à stocker pour réguler la température de stockage entre le jour et la nuit, été comme hiver

On appelle matériau à changement de phase - ou MCP - tout matériau capable de changer d'état physique (généralement fusion/solidification) à température quasi constante, appelée température de changement de phase, tout en stockant une grande quantité d'énergie. La température de changement de phase est avantageusement située entre 10 et 80 degrés.

À cette fin, la présente invention a pour objet un boîtier thermo régulé autonome comprenant, par référence à la position d'utilisation :
- Une chambre de stockage, recouverte d'une couche de matériau isolant thermique, et munie d'un support pour un produit à stocker;
- Une chambre de régulation, séparée de la chambre de stockage par une cloison munie de deux orifices de passage d'air, et comprenant
   - une paroi de canalisation fixée entre les deux orifices de passage d'air, délimitant un circuit de circulation d'air avec deux colonnes de circulation d'air et un passage entre ces deux colonnes ;
   - un volume déterminé d'un matériau à changement de phase ;
- Un moyen de chauffage du matériau à changement de phase

Contrairement aux systèmes antérieurs, le boîtier selon l'invention présente une totale autonomie et n'a pas de contrainte de rechargement à une source d'énergie externe autres que les sources d'énergie renouvelables directement disponible dans son environnement immédiat. Il peut donc être installé de manière très polyvalente.

Selon d'autres modes de réalisation, qui peuvent être combinés entre eux :
- la chambre de régulation peut être agencée sous la chambre de stockage ;
- la chambre de régulation peut être agencée latéralement par rapport à la chambre de stockage ;
- la chambre de régulation peut être agencée au-dessus de la chambre de stockage ;
- la chambre de régulation peut être délimitée par une paroi conductrice thermique ;
- la chambre de régulation peut être munie d'un vitrage sur une face destinée à être exposée au soleil, en position d'utilisation, et dans lequel le volume de matériau à changement de phase est recouvert d'une plaque de capture de chaleur agencée en regard du vitrage, le vitrage et la plaque de capture de chaleur constituant un moyen de chauffage du matériau à changement de phase ;
- le volume de matériau à changement de phase peut être supporté par la paroi de canalisation ;
- le volume de matériau à changement de phase peut être supporté par une paroi de la chambre de régulation ;
- le volume déterminé de matériau à changement de phase peut être fixé à distance de la paroi qui le porte, de manière à ménager un double circuit de circulation d'air de part et d'autre du volume de matériau à changement de phase ;
- le matériau à changement de phase peut être combiné à un matériau conducteur de chaleur divisé ;
- le boîtier peut comprendre, en outre, au moins un panneau de cellules photovoltaïques relié à une batterie elle-même reliée à un microcontrôleur apte à commander le fonctionnement d'au moins un équipement électrique incorporé au boîtier ;
- le microcontrôleur peut être apte à commander le fonctionnement d'une résistance électrique en contact thermique avec le volume de matériau à changement de phase ;
- le microcontrôleur peut être relié à un émetteur/récepteur de liaison sans fil à faible consommation énergétique, capable d'émettre et de recevoir des données sur un réseau étendu sans fil à faible consommation énergétique, dit « LPWAN » et/ou sur un réseau local sans fil à faible consommation énergétique, dit « LPLAN » ;
- le boîtier peut comprendre, en outre, au moins un ventilateur commandé par le microcontrôleur ;
- le boîtier peut comprendre, en outre, au moins un équipement électronique de contrôle relié au microcontrôleur ;
- l'équipement électronique de contrôle peut être choisi parmi une sonde de température, un capteur d'hygrométrie, un capteur d'état de l'objet stocké, un contact sec, ou un assortiment de ceux-ci ;
- les orifices de passage d'air peuvent être équipés chacun d'une trappe commandable entre une position ouverte autorisant un passage d'air et une position fermée bloquant le passage de l'air ;
- le boîtier peut comprendre, en outre, des orifices d'entrée/sortie d'air commandables entre une position ouverte autorisant un passage d'air de ou vers l'extérieur du boîtier et une position fermée bloquant le passage de l'air ; et/ou
- la chambre de stockage peut comprendre deux orifices d'entrée/sortie d'air, et la chambre de régulation comprend un orifice d'entrée/sortie d'air.

D'autres caractéristiques de l'invention seront énoncées dans la description détaillée ci-après, faite en référence aux dessins annexés, qui représentent, respectivement :
- les figures 1 et 2, des vues schématiques en coupe de deux variante d'un premier mode de réalisation d'un boîtier selon l'invention en période d'été, respectivement le jour et la nuit ;
- les figures 3 et 4, des vues schématiques en coupe d'une variante du mode de réalisation des boîtiers des figures 1 et 2, en période d'hiver, respectivement le jour et la nuit, et muni d'un système de surveillance à distance et d'un moyen de chauffage commandé du matériau à changement de phase ;
- la figure 5, une vue schématique en coupe d'un deuxième mode de réalisation d'un boitier selon l'invention, dans lequel le volume de matériau à changement de phase est fixé contre la paroi extérieure de la chambre de régulation ;
- les figures 6 et 7, des vues schématiques en coupe d'un troisième mode de réalisation, respectivement lorsque le boîtier emmagasine de la chaleur extérieure et lorsque le boîtier redistribue de la chaleur au produit en raison des fluctuations de température extérieures durant l'exploitation d'un boîtier selon l'invention muni d'un système de chauffage «Trombe» du matériau à changement de phase ;
- la figure 8, une vue schématique en coupe d'une première variante de boîtier des figures 6 et 7, muni d'un volume de matériau à changement de phase fixé au système de chauffage « Trombe » ;
- Les figure 9 à 12, des vues schématiques en coupe du boîtier de la figure 8, dans différentes situations d'utilisation ;
- La figure 13, une vue schématique en coupe d'un sixième mode de réalisation d'un boitier selon l'invention, dans lequel la chambre de régulation est agencée latéralement par rapport à la chambre de stockage ; et
- La figure 14, une vue schématique en coupe d'un septième mode de réalisation d'un boitier selon l'invention, dans lequel la chambre de régulation est agencée au-dessus de la chambre de stockage.

L'invention est décrite ci-après avec un moyen de chauffage utilisant l'énergie solaire. Cependant, il est également possible d'utiliser un moyen de chauffage utilisant la géothermie de surface dans les lieux adaptés.

Le mode de réalisation illustré est particulièrement adapté à un boîtier de type « boîtier pour défibrillateur externe » sur pied.

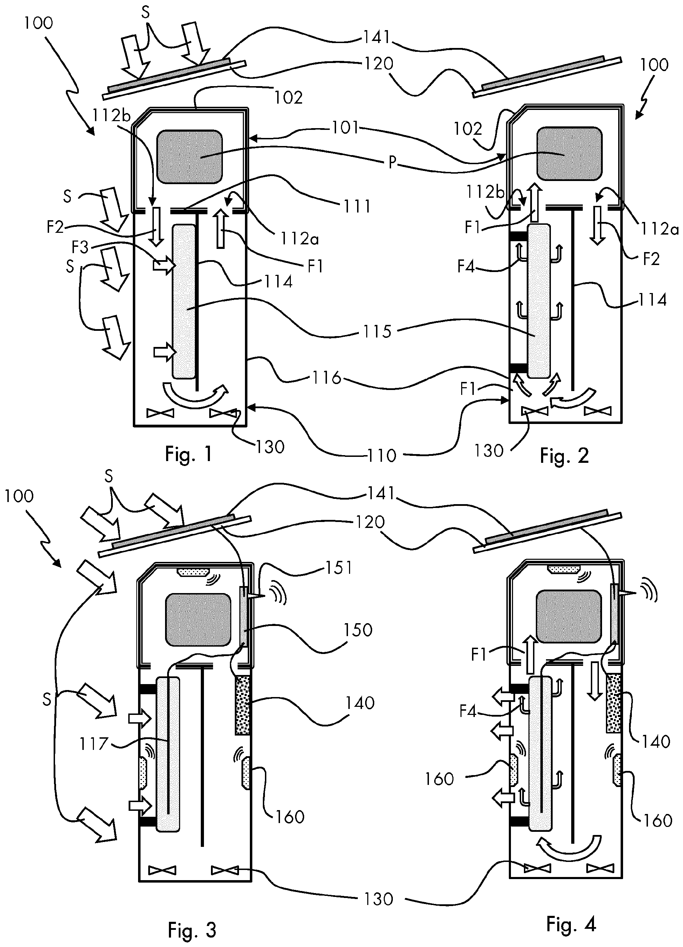

Le boîtier 100 illustré en figure 1 comprend, par référence à la position d'utilisation illustrée, une chambre de stockage supérieure 101, recouverte d'une couche 102 de matériau isolant thermique à faible conductivité et haute résistance thermique pour isoler thermiquement l'intérieure de la chambre de stockage et éviter les gains/déperditions de chaleur dus aux écarts de températures journaliers et saisonniers.

La chambre de stockage 101 est munie d'un support (non illustré par souci de clarté) pour le produit à stocker P, agencé de telle sorte que de l'air puisse circuler autour de l'objet P.

Le boîtier 100 selon l'invention illustré dans cet exemple comprend également une chambre de régulation inférieure 110, séparée de la chambre de stockage 101 par une cloison 111, ici horizontale, munie de deux orifices de passage d'air 1 12a- et 1 12b.

Une paroi de canalisation 1 14 est fixée entre les deux orifices de passage d'air par une seule extrémité, délimitant un circuit de circulation d'air avec une colonne de montée d'air et une colonne de descente d'air et un passage entre ces deux colonnes, en bas de la chambre de régulation 110 dans la configuration illustrée. À cette fin, la paroi de canalisation 114 est de longueur inférieure à la longueur de la chambre de régulation 110 pour laisser passer l'air entre la colonne de montée d'air et la colonne de descente d'air. Dans une variante non illustrée, la paroi de canalisation 114 peut séparer totalement la chambre de régulation en deux et être fixée par deux extrémités à ladite chambre. Dans ce cas, elle doit comporter un orifice suffisamment grand pour permettre une circulation d'air entre les deux colonnes de circulation d'air ainsi ménagées.

Selon l'invention, la chambre de régulation 110 comprend également un volume déterminé d'un matériau à changement de phase 115.

Avantageusement, le matériau à changement de phase choisi est de type paraffines car elles ne présentent pas de perte ou de modification de leurs caractéristiques physiques liées à leur vieillissement. Le boîtier selon l'invention peut donc rester opérationnel sur une durée pluriannuelle sans perte d'efficience qui serait due à un vieillissement des propriétés réactives du matériau à changement de phase.

L'autre avantage est que la température de changement de phase des paraffines est compatible avec les températures été/hiver de très nombreux pays.

La conductivité thermique du matériau à changement de phase étant souvent faible, elle peut être avantageusement augmentée par l'ajout d'un matériau conducteur de chaleur divisé tel que le graphite. On entend par matériau conducteur de chaleur divisé un matériau conducteur de chaleur particulaire, par exemple une poudre ou un ensemble de paillettes.

Le composite ainsi mis en oeuvre permet d'obtenir les niveaux de puissance de charge et décharge thermique souhaités. Ce paramètre de conductivité doit être optimisé en fonction de la quantité de matériau à changement de phase utilisé, de la surface d'échange entre le matériau à changement de phase et l'air ventilé, de la différence de température entre l'air et le matériau à changement de phase et de la vitesse d'écoulement de l'air ventilé.

La combinaison du matériau à changement de phase avec un matériau conducteur de chaleur divisé peut être faite par mélange du matériau à changement de phase et du matériau conducteur de chaleur divisé. Alternativement, le matériau conducteur de chaleur divisé peut être contenu dans une enveloppe conductrice de chaleur en contact thermique avec le matériau à changement de phase et absorbant l'énergie solaire.

Selon l'invention, le matériau à changement de phase et/ou le mélange de plusieurs matériaux à changement de phase et/ou le mélange d'un ou plusieurs matériau(x) à changement de phase avec un matériau conducteur de chaleur divisé de type graphite, sont choisi pour que la température de changement de phase soit entre la température minimale et la température maximale de la gamme de température dans laquelle doit être stocké l'objet P.

Selon l'invention, on choisit le matériau à changement de phase (ou un mélange) qui présente une température de changement de phase (fusion/liquéfaction) sensiblement égale à la moyenne de la gamme de température de stockage : si cette dernière est comprise entre 15°C et 45°C, on peut choisir un matériau à changement de phase (ou un mélange) dont la température de changement de phase est sensiblement égale à 30°C.

Ainsi, le matériau à changement de phase n'est pas choisi pour que sa température de changement de phase soit égale à la température maximale ou la température minimale de la gamme de température de stockage de consigne de l'objet.

Selon l'invention, le boîtier 100 comprend également un moyen de chauffage du matériau à changement de phase.

Dans le mode de réalisation illustré en figure 1, la chambre de régulation 110 est délimitée par une paroi extérieure 116 conductrice thermique. Cette dernière transmet donc la chaleur au matériau à changement de phase à travers la colonne d'air, par convection et/ou rayonnement.

Cependant, ces transferts thermiques ne sont pas les plus efficaces. Un mode de réalisation avantageux illustré en figure 2, le volume déterminé de matériau à changement de phase est fixé à distance de la paroi qui le porte, de manière à ménager un double circuit de circulation d'air de part et d'autre du volume de matériau à changement de phase.

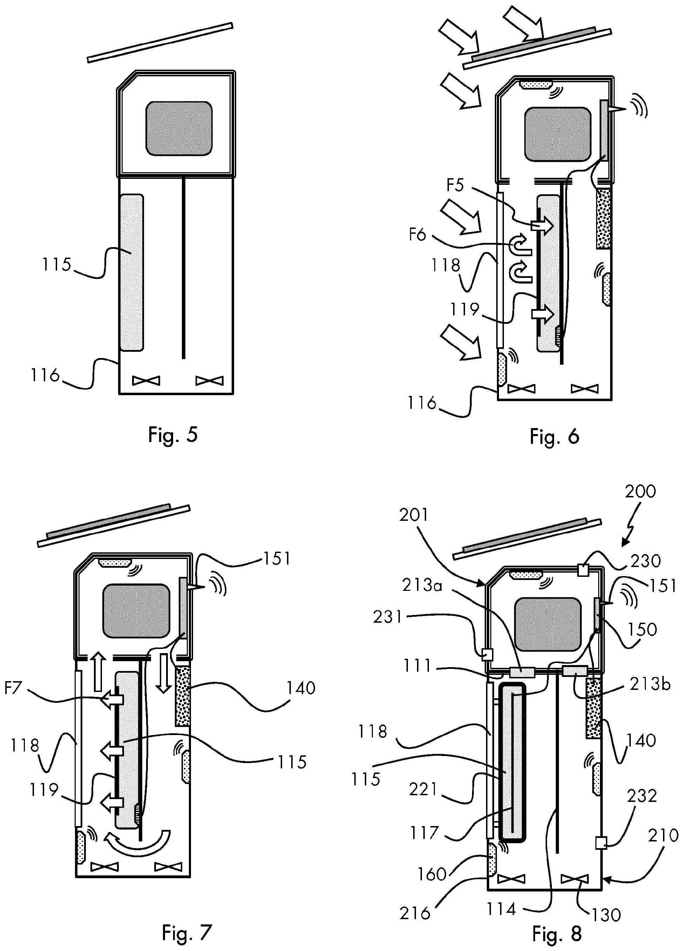

Dans le mode de réalisation illustré, le volume de matériau à changement de phase est avantageusement supporté par une paroi 116 de la chambre inférieure de régulation 110, de préférence par des fixations 115a conductrices de chaleur. Un autre mode de réalisation est illustré en figure 5, dans lequel le volume de matériau est changement de phase est fixé en contact thermique avec la paroi 116, ce qui favorise son réchauffement pendant la journée.

En été, la température haute extrême est atteinte le jour et il convient globalement de maintenir l'objet au frais.

Le jour (figure 1), le boîtier 100 est réchauffé au cours de la journée, et cette chaleur chauffe l'air contenu dans le boîtier par l'intermédiaire de la paroi extérieure 116 conductrice de chaleur de la chambre de régulation 110.

Ceci génère une circulation naturelle d'air : l'air chaud remonte (flèche F1) et l'air frais descend (flèche F2).

L'air chaud transmet sa chaleur au matériau à changement de phase 115 (flèche F3) qui se liquéfie au fur et à mesure. Ce changement de phase se fait à température constante, de sorte que l'objet P, structurellement protégé d'un effet potentiel de surchauffe par la couche isolante 102, est également maintenu à une température proche de la température de changement de phase grâce à la circulation d'air tempéré qui a cédé sa chaleur au matériau à changement de phase 115.

Ainsi, la chaleur à évacuer au niveau de l'objet P est stockée par le matériau à changement de phase 115 par fusion de ce dernier. En d'autres termes, la température de changement d'état du matériau à changement de phase empêche l'objet P de monter en température.

Pendant la nuit (figure 2), lorsque la température extérieure baisse, la paroi extérieure 116 de la chambre de régulation refroidit. Même si cette température devient trop basse vis à vis du produit P, la chaleur dégagée lors du changement de phase (solidification) du matériau à changement de phase 115 maintient l'air intérieur du boîtier à une température tempérée (flèches F4), et ce jusqu'à complète solidification.

L'air environnant extérieur permet ainsi de solidifier le matériau à changement de phase 115 qui est alors à nouveau disponible pour protéger le contenu le jour suivant en rafraîchissant l'air intérieur du boîtier.

Le volume de matériau à changement de phase est donc choisi selon les températures du lieu géographique pour que cette solidification totale n'arrive qu'en toute fin de nuit, lorsque la température commence à remonter, et que la liquéfaction totale n'arrive qu'en fin d'après-midi, lorsque la température commence à baisser.

Ainsi, le matériau à changement de phase 115 est utilisé pour tempérer le produit P par ventilation d'air entre les deux chambres 101 et 110.

La chambre de régulation 110 est ainsi équipée d'un circuit de circulation d'air assurant les transferts thermiques entre l'objet P, placé dans la chambre de stockage supérieure, et le matériau à changement de phase 115. La nuit, lorsque l'objet P est à réchauffer par le matériau à changement de phase 115, l'air est ventilé par convection naturelle. Le jour, lorsque le contenu est à refroidir par le matériau à changement de phase 115, l'air est avantageusement mis en mouvement par des ventilateurs 130 pour forcer la ventilation d'air à l'intérieur du boîtier, et commandés par un microcontrôleur.

Ces ventilateurs n'ont pas besoin d'être très puissants, de sorte qu'ils ne consomment que très peu d'énergie. Pour les alimenter, le boitier selon l'invention comprend avantageusement un panneau 120 recouvert de cellules photovoltaïques 141 reliées électriquement aux ventilateurs 130 (connexion non illustrée par souci de clarté des figures). Alternativement, ils peuvent être alimentés par des piles présentant plusieurs années de durée de vie.

Plus précisément, les cellules photovoltaïques 141 sont reliées à une batterie 140 qui alimente électriquement un microcontrôleur 150 commandant le fonctionnement d'équipements électriques compris dans le boîtier, notamment le ou les ventilateurs 130.

Les figures 3 et 4 illustrent le fonctionnement où la température basse extrême est atteinte la nuit, par exemple en hiver. Il convient donc globalement de maintenir l'objet P au chaud. Ceci est obtenu grâce au moyen de chauffage du boîtier 100 selon l'invention.

Dans le mode de réalisation illustré, le chauffage par conduction par la paroi 116 est complété par une résistance électrique 117 en contact thermique avec le matériau à changement de phase 115, ce qui améliore le rendement du chauffage du matériau à changement de phase. Cette résistance électrique 117 est reliée au microcontrôleur 150 et alimentée par les cellules photovoltaïques 141. Le microcontrôleur 150 commande avantageusement le fonctionnement de la résistance électrique en fonction de données de température qu'il récupère de sondes de température 160 disposés à différents endroits du boîtier.

Au cours de la journée (figure 3), le soleil peut atteindre la chambre de stockage 101 et la réchauffer. De même le soleil réchauffe la chambre de régulation 110 grâce à la paroi extérieure 116 conductrice de chaleur.

Si la température extérieure est suffisante pour liquéfier le matériau à changement de phase 115, ce dernier absorbe de la chaleur en changeant de phase, énergie qu'il restituera la nuit lorsque la température baissera.

Cependant, en général, la température extérieure du jour est insuffisante, en hiver, pour liquéfier le matériau à changement de phase 115.

Selon l'invention, la résistance électrique alimentée par les cellules photovoltaïques permet de chauffer par effet de Joule le matériau à changement de phase et de liquéfier, pendant la journée. Le matériau à changement de phase retransmettra pendant la nuit de la chaleur à l'objet en retournant à l'état solide.

Ainsi, le jour (figure 3), la résistance électrique alimentée par les cellules photovoltaïques chauffe le matériau à changement de phase 115 qui se liquéfie. Le matériau à changement de phase 115 est alors disponible pour tempérer l'air intérieur du boîtier pendant la nuit.

Ainsi, la nuit (figure 4), lorsque la température extérieure baisse suffisamment pour que l'air intérieur du boîtier soit en dessous de la température de changement de phase, le matériau à changement de phase 115 se solidifie, restituant de la chaleur à l'air. Ce dernier monte alors naturellement vers l'objet P stocké (flèche F1).

Les figures 6 à 13 illustrent plusieurs variantes du mode de réalisation préféré d'un moyen de chauffage du matériau à changement de phase, présentant un rendement optimal.

Dans ce mode de réalisation, la chambre de régulation 110 est munie d'un vitrage 118 sur la face de la chambre 110 qui est exposée au soleil en position d'utilisation.

En regard du vitrage 118, le boîtier selon l'invention comprend une plaque de capture de chaleur 119 recouvrant le volume de matériau à changement de phase 115. Alternativement ou en combinaison, le matériau à changement de phase peut être recouvert d'une enveloppe conductrice de chaleur peinte en noir 221 (voir figure 8).

Le vitrage 118 et la plaque de capture de chaleur 119 constituent un système de chauffage de type «Trombe» du matériau à changement de phase 115.

Le système de chauffage de type « Trombe » disposé sur le boîtier 100 permet de capter le rayonnement solaire et de le convertir en chaleur : la surface noire de la plaque 119 ou de l'enveloppe 221 absorbe le rayonnement solaire, le converti en chaleur directement et le transfert au matériau à changement de phase 115 (flèches F5). En outre, la surface noire rayonne dans l'infrarouge et ce rayonnement bloqué par effet de serre par le vitrage (flèche F6) est utilisé en partie pour réchauffer directement l'objet P et pour le reste à stocker de la chaleur dans le matériau à changement de phase 115 en le liquéfiant. Ce dernier est alors fonctionnel pour la nuit (figure 7), durant laquelle il va se solidifier au fur et à mesure en restituant de la chaleur à l'air intérieur de la chambre de régulation 110 (flèches F7). Un mouvement de convection naturel s'installe alors, et l'air chaud remonte dans la chambre de stockage 101 pour maintenir l'objet P à une température de stockage prescrite.

Dans la variante des figures 6 et 7, le volume de matériau à changement de phase est supporté par la paroi de canalisation 114. Dans une variante non illustrée, le volume déterminé de matériau à changement de phase est fixé à distance de la paroi de canalisation 114, de manière à ménager un double circuit de circulation d'air de part et d'autre du volume de matériau à changement de phase.

Les meilleurs rendements sont obtenus lorsque le volume de matériau à changement de phase 115 est supporté par une paroi 116 de la chambre inférieure de régulation 110, en regard et à distance du vitrage 118, de manière à ménager un double circuit de circulation d'air de part et d'autre du volume de matériau à changement de phase. Cette variante est illustrée aux figures 8 à 13. Dans ce mode de réalisation, la paroi 116 qui délimite la chambre de régulation n'est pas conductrice thermique.

Le mode de réalisation illustré en figures 8 comprend plusieurs perfectionnements qui peuvent être avantageusement combinés individuellement aux modes de réalisation précédemment décrits.

Ainsi, la chambre de régulation 210 du boîtier 200 comprend une paroi 216 munie d'un vitrage 118 sur une face destinée à être exposée au soleil. Le volume déterminé de matériau à changement de phase 115 est fixé à distance de la paroi 216 qui le porte, de manière à ménager un double circuit de circulation d'air de part et d'autre du volume de matériau à changement de phase. Cet agencement préféré maximise le rendement d'échange thermique et le fonctionnement du boîtier.

Le volume déterminé de matériau à changement de phase 115 est également recouvert d'une plaque de capture de chaleur 221, ici sous forme d'une enveloppe complète, mais seule la face agencée en regard du vitrage pourrait être seules recouverte.

Ce mode de réalisation illustre également la possibilité avantageuse d'équiper chacun des orifices de passage d'air séparant la chambre de régulation 210 de la chambre de stockage 201 avec une trappe commandable, respectivement 213a et 213b entre une position ouverte autorisant un passage d'air et une position fermée bloquant le passage de l'air. Sur les figures, la position fermée est illustrée par un rectangle barré et la position ouverte par un rectangle plein.

Concomitamment, le boîtier 200 est équipé de ventilateurs 130 commandables par le microcontrôleur 150 afin de générer des flux d'air dans une direction souhaitée. Les ventilateurs peuvent être inversés pour inverser la direction du flux d'air.

Dans un mode de réalisation non illustré, les trappes commandables 213a et 213b sont elles mêmes équipées de ventilateurs inversables, ce qui rend les échanges d'air entre la chambre de stockage et la chambre de régulation plus efficaces et plus précis.

Un autre perfectionnement particulièrement utile est la présence d'orifices d'entrée/sortie d'air commandables entre l'intérieur et l'extérieur du boîtier 200 : deux orifices d'entrée/sortie d'air 230 et 231 dans la chambre de stockage, et un orifice d'entrée/sortie d'air 232 dans la chambre de régulation.

De préférences, les orifices d'entrée/sortie d'air 230 et 231 sont agencées de part et d'autre de l'objet stocké et de son support de manière à favoriser une circulation d'air autour de l'objet.

Sur les figures, la position fermée est illustrée par un rectangle barré et la position ouverte par un rectangle plein.

Les trappes commandables et les orifices d'entrée/sortie d'air commandables sont soit commandées électriquement par le microcontrôleur, soit commandée mécaniquement en fonction des conditions de température.

Comme précédemment décrit, le matériau à changement de phase est avantageusement combiné avec un matériau conducteur de chaleur divisé, tel que du graphite.

Le matériau à changement de phase 115 est également avantageusement en contact thermique avec une résistance électrique 117 alimentée par le microcontrôleur 150 en fonction des conditions de température et d'humidité captées par différents équipements électroniques de contrôle 160 incorporés au boîtier, par exemple : une sonde de température, un capteur d'hygrométrie, un capteur d'état de l'objet stocké (Par exemple un capteur optique servant à contrôler l'état de marche d'un défibrillateur stocké ou un capteur de gaz), un contact sec, ou un assortiment de ceux-ci.

L'ensemble est alimenté par un panneau de cellules photovoltaïques 141 relié à une batterie 140 elle-même reliée au microcontrôleur 150.

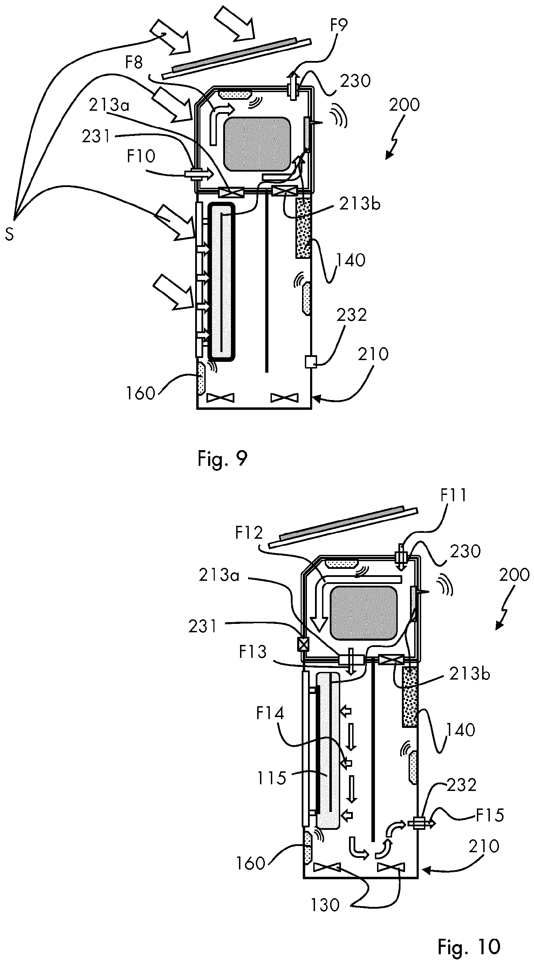

La figure 9 illustre le fonctionnement du boîtier de la figure 8 lors de situations différentes.

Il peut s'agir, par exemple, d'une journée d'été pendant laquelle le boîtier reçoit un rayonnement S. dans ce cas, la température intérieure du boitier augmente et devient supérieure à la température de l'air extérieur. L'objectif est donc de limiter l'échauffement de l'objet.

Lorsque la température intérieure du boîtier est supérieure à une température seuil donnée (par exemple la température extérieure), les trappes 213a et 213b se ferment, et les orifices d'entrée/sortie d'air 230 et 231 s'ouvrent pour permettre une circulation d'air extérieur autour de l'objet (flèche F8) dans la chambre de stockage 201 par convection naturelle.

En effet, l'invention prévoit que l'orifice d'entrée/sortie 231 est agencé à une hauteur inférieure que l'orifice d'entrée/sortie 230. L'air chaud (réchauffé par le rayonnement du soleil) sort donc par ce dernier (flèche F9), et l'air plus frais de l'extérieur entre par l'orifice 231 (flèche F10). En outre, les orifices d'entrée/sortie 230-231 sont agencé de manière opposée par rapport à l'objet, forçant ainsi le passage de l'air autour de l'objet.

Dans cette situation, la chambre de régulation est isolée de la chambre de stockage car les trappes 213a et 213b sont fermées.

Le rayonnement lumineux passe au travers du vitrage et chauffe le matériau à changement de phase, comme expliqué précédemment. Ce dernier emmagasine donc de la chaleur qu'il pourra retransmettre plus tard.

Cette situation a également lieu lors d'une journée d'hiver, lorsque le rayonnement chauffe fortement la chambre de stockage mais que l'air extérieur est frais. Dans ce cas, le rayonnement charge le matériau à changement de phase en chaleur en vue de la nuit (pour réchauffer l'objet). Le jour, l'objet est refroidi par la circulation d'air frais extérieur.

Cette situation a également lieu lorsque la température extérieure est supérieure à la température du changement de phase du matériau à changement de phase. Dans ce cas, l'échange thermique direct par circulation d'air est plus efficace qui l'échange thermique via le matériau à changement de phase de la chambre de régulation.

La figure 10 illustre le fonctionnement du boîtier de la figure 8, par exemple lors d'une nuit d'été : pas de rayonnement solaire et température qui baisse. L'objectif reste de rafraîchir l'objet.

Dans ce cas, l'orifice d'entrée/sortie supérieur 230 est ouverte, alors que l'orifice d'entrée/sortie inférieure 231 est fermée. L'orifice d'entrée/sortie 232 de la chambre de régulation, agencé dans la colonne d'air opposée à la colonne d'air dans laquelle est située le volume de matériau à changement de phase, est ouvert.

La trappe 213a, débouchant dans la colonne d'air dans laquelle est située le volume de matériau à changement de phase, est ouverte, alors que la trappe 213b débouchant dans la colonne d'air opposée à la colonne d'air dans laquelle est située le volume de matériau à changement de phase, est fermée.

La circulation d'air est forcée par l'actionnement des ventilateurs 130, de sorte que l'air extérieur entre dans le boîtier par l'orifice 230 (flèche F11), circule autour de l'objet en le rafraîchissant (flèche F12), et pénètre dans la chambre de régulation par la trappe 213a (flèche F13). les ventilateurs sont alimentés par la batterie chargée au cours de la journée par les panneaux solaires

Au contact du matériau à changement de phase 115, l'air extérieur rafraîchit le matériau à changement de phase (flèche F14) qui change de phase en se solidifiant. Ce faisant, il réchauffe l'air qui est évacué par l'orifice 232 (flèche F15) car il ne peut remonter vers l'objet puisque la trappe 213b est fermée.

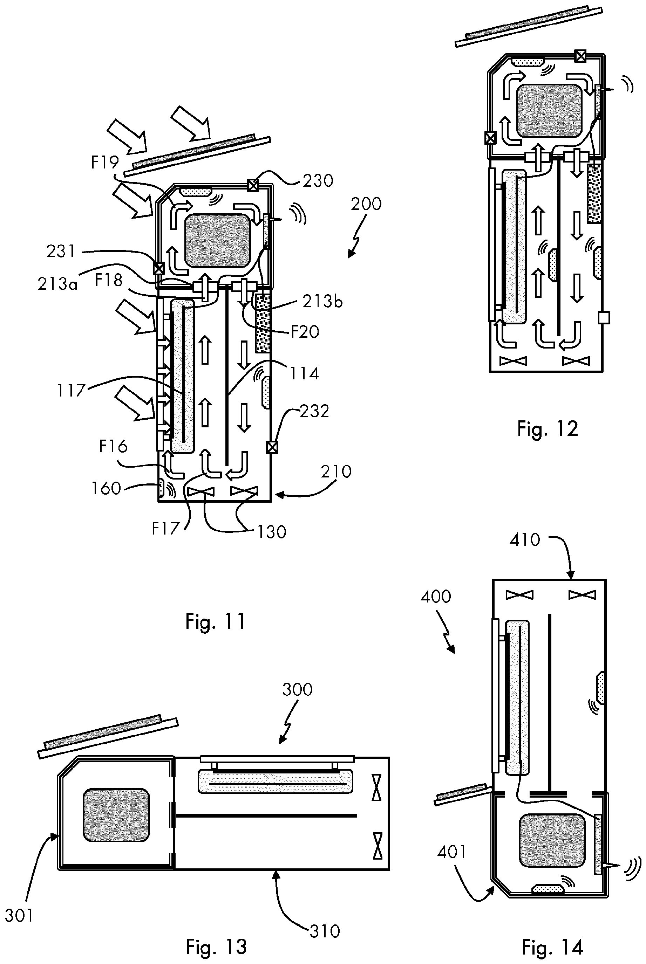

Les figures 11 et 12 illustrent le fonctionnement du boîtier de la figure 8 pendant la période froide, au cours de laquelle l'objectif est de réchauffer l'objet.

La figure 11 illustre le fonctionnement pendant la journée au cours de laquelle le boîtier reçoit un rayonnement lumineux.

Pour limiter le refroidissement de l'objet, les orifices d'entrée/sortie 230, 231 et 232 sont fermés.

En période froide, la température extérieure la journée reste inférieure à la température de changement de phase du matériau à changement de phase.

Le rayonnement lumineux transmis par le vitrage réchauffe le matériau à changement de phase qui emmagasine de la chaleur en changeant de phase. L'air se réchauffe au contact du matériau à changement de phase : entre le matériau à changement de phase et le vitrage (flèche F16), et entre le matériau à changement de phase et la paroi de canalisation 114 (flèche F17).

L'air chaud remonte naturellement et entre dans la chambre de stockage par la trappe 213a qui est ouverte (flèche F18).

L'air chaud circule autour de l'objet et le garde en température (flèche F19).

L'air se refroidit au contact de l'objet et redescend dans la chambre de régulation par la trappe 213b ouverte (flèche F20), débouchant dans la colonne d'air opposée à la colonne d'air dans laquelle est située le volume de matériau à changement de phase.

Si le rayonnement lumineux est un peu faible, l'apport de chaleur au matériau à changement de phase peut être complété par l'alimentation de la résistance 117. Alternativement ou en combinaison, on peut utiliser un matériau à changement de phase combiné à un matériau conducteur de chaleur divisé afin d'améliorer le rendement de transfert de chaleur et obtenir la puissance de stockage/déstockage voulue.

En cas d'excès de chaleur dans la chambre de stockage, les orifices d'entrée/sortie230-231 peuvent être ouverts temporairement.

La figure 12 illustre le fonctionnement pendant la nuit au cours de laquelle le boîtier ne reçoit plus aucun rayonnement lumineux.

Dans ce cas, la convection naturelle amorcée pendant la journée se poursuit pendant la nuit, alimentée par le réchauffement de l'air lors du changement de phase du matériau à changement de phase.

Dans les modes de réalisation précédents, l'agencement supérieur de la chambre de stockage par rapport à la chambre de régulation en position inférieure permet une convection naturelle de l'air dans certaines conditions de température. Cet agencement est particulièrement avantageux lorsqu'il est possible d'installer un tel boîtier.

Cependant, dans certains cas, il peut être souhaitable de modifier un coffre préexistant stockant un objet devant être thermo-régulé afin, par exemple, de diminuer la consommation d'énergie utilisée pour cette régulation thermique.

Il n'est alors pas possible à des coûts raisonnables d'insérer une chambre de régulation selon l'invention sous le coffre préexistant pour lui donner la fonction de chambre de stockage selon l'invention.

La figure 13 illustre un premier agencement possible pour former un boîtier 300 selon l'invention à partir d'une chambre de stockage préexistante 301.

Dans cet agencement, une chambre de régulation 310 selon l'invention est agencée latéralement par rapport à la chambre de stockage 301.

La figure 14 illustre un deuxième agencement possible pour former un boîtier 400 selon l'invention à partir d'une chambre de stockage préexistante 401.

Dans cet agencement, une chambre de régulation 410 selon l'invention est agencée au-dessus par rapport à la chambre de stockage 401.

Lors de l'adaptation, il faut ouvrir deux orifices de passage d'air entre la chambre de régulation et la chambre de stockage, puis installer l'ensemble équipement électriques et électroniques. Avantageusement, on percera également la chambre de stockage avec des orifices d'entrée/sortie d'air.

Dans tous les modes de réalisation précédents, le fonctionnement et/ou le contrôle à distance peut/peuvent être assuré(s) grâce au microcontrôleur 150 et à des capteurs électroniques 160, tels que des sondes de températures ainsi qu'à des capteurs d'hygrométrie et différents contacts sec, disposées au niveau de chaque élément constitutif du boîtier selon l'invention (matériau à changement de phase 115, colonnes d'air, chambre de stockage 101, surface extérieur).

Le boîtier selon l'invention comprend également avantageusement un module de télécommunication permettant notamment de superviser à distance les températures relevées à chaque niveau des composants par les capteurs 160 de température.

À cette fin, le microcontrôleur 150 est avantageusement équipé d'un système de communication comprenant un émetteur/récepteur 151 de liaison sans fil, de préférence de type LRWA (Long Range Wild Area) à basse consommation énergétique.

Ce type d'émetteur récepteur 151 est capable d'émettre et de recevoir des données sur un réseau étendu sans fil à faible consommation énergétique, dit « LPWAN » et/ou sur un réseau local sans fil à faible consommation énergétique, dit « LPLAN ».

Par exemple, l'émetteur/récepteur 151 est capable d'émettre et de recevoir selon les protocoles de communication éprouvés et fréquemment utilisés dans les réseaux dits de type « loT » . Ces modes de communication sont bas débit, de longue portée et très peu énergivores.

Avantageusement, une carte électronique spécifiquement adaptée à la surveillance du contenu et communicant au travers des réseaux LRWA peut garantir un suivi permanent de l'environnement et de son état.

L'utilisation de ces réseaux à très basse consommation électrique permet un fonctionnement autonome soit au travers de capacités elles-mêmes alimentées par les cellules photovoltaïques, soit au travers de piles ou batteries.

Dans le cas de l'option de surveillance et de contrôle à distance, un serveur de type « cloud » permet de superviser un ensemble de boîtiers selon l'invention, géolocalisés sur une carte, et d'accéder individuellement à chacun des boîtiers et du relevé des données issues de l'ensemble des équipements électroniques de contrôle 160 tels que les sondes de température, les capteurs hygrométriques et les contacts sec stockés sur une mémoire (non illustrée) reliés au microcontrôleur 150.

La combinaison d'un isolant à haute résistance thermique et faible conductivité associé à un volume de matériau à changement de phase spécifiquement adapté au niveau des températures recherchées, l'ensemble couplé à des échanges thermiques avec l'environnement extérieur immédiat, à un moyen de chauffage du matériau à changement de phase (système trombe ou résistance électrique alimentée par des cellules photovoltaïques) et un circuit de ventilation, permet d'assurer au boîtier des températures régulées avec de faibles écarts de variations sur des plages de 24 heures.

La solution selon l'invention permet de s'affranchir de toutes sources d'énergies extérieures au boîtier autres que son environnement extérieur immédiat et sources d'énergies renouvelables telle que le solaire, et d'éviter l'utilisation d'énergies fossiles ou de matériaux chimiques à recharger.

L'invention permet au dispositif d'être protégé des contraintes en températures (variations) rencontrées entre le jour et la nuit mais aussi été comme hiver (lorsque le contenu est limité en température haute et basse).

Un tel dispositif permet de s'affranchir des besoins en recharge d'énergie et reste opérationnel sur une durée pluriannuelle sans perte d'efficience.

Il est alors possible de stocker un objet, tel qu'un défibrillateur, de manière sûr, y compris dans des endroits non raccordés au réseau électrique.

Les dimensions du boîtier et de ses composants, le choix du matériau à changement de phase et l'ajout éventuel d'un matériau conducteur de chaleur divisé et/ou d'une résistance électrique, peuvent varier en fonction du produit à stocker (la nature de l'énergie renouvelable sollicitée peut varier aussi: on peut utiliser le solaire et/ou la géothermie de surface).

## Revendications

1. Boîtier thermo régulé autonome (100, 200, 300, 400) comprenant :
• Une chambre de stockage (101, 201, 301, 401), recouverte d'une couche de matériau isolant thermique (102), et munie d'un support pour un produit (P) à stocker ;
• Une chambre de régulation (110, 210, 310, 410), séparée de la chambre de stockage (101, 201, 301, 401) par une cloison (111) munie de deux orifices de passage d'air (1 12a-1 12b, 213a-213b), et comprenant
- une paroi de canalisation (114) fixée entre les deux orifices de passage d'air (112a-112b, 213a-213b), délimitant un circuit de circulation d'air avec deux colonnes de circulation d'air et un passage entre ces deux colonnes ;
- un volume déterminé d'un matériau à changement de phase (115) ;
**caractérisé par**
• Un moyen de chauffage (116, 117, 118-119, 221) du matériau à changement de phase (115).

2. Boîtier selon la revendication 1, dans lequel, par référence à la position d'utilisation, la chambre de régulation (110) est agencée sous la chambre de stockage (101).

3. Boîtier selon la revendication 1, dans lequel, par référence à la position d'utilisation, la chambre de régulation (310) est agencée latéralement par rapport à la chambre de stockage (301).

4. Boîtier selon la revendication 1, dans lequel, par référence à la position d'utilisation, la chambre de régulation (410) est agencée au-dessus de la chambre de stockage (401).

5. Boîtier selon la revendication 1, dans lequel la chambre de régulation (110) est délimitée par une paroi conductrice thermique (116).

6. Boîtier selon l'une quelconque des revendications 1 à 5, dans lequel la chambre de régulation (110, 210) est munie d'un vitrage (118) sur une face destinée à être exposée au soleil, en position d'utilisation, et dans lequel le volume de matériau à changement de phase (115) est recouvert d'une plaque de capture de chaleur (119, 221) agencée en regard du vitrage (118), le vitrage et la plaque de capture de chaleur constituant un moyen de chauffage du matériau à changement de phase.

7. Boîtier selon l'une quelconque des revendications 1 à 6, dans lequel le volume de matériau à changement de phase (115) est supporté par la paroi de canalisation (114).

8. Boîtier selon l'une quelconque des revendications 1 à 6, dans lequel le volume de matériau à changement de phase (115) est supporté par une paroi extérieure (116, 216) de la chambre inférieure de régulation (110).

9. Boîtier selon l'une quelconque des revendications 7 ou 8, dans lequel le volume déterminé de matériau à changement de phase (115) est fixé à distance de la paroi (114, 116, 216) qui le porte, de manière à ménager un double circuit de circulation d'air de part et d'autre du volume de matériau à changement de phase.

10. Boîtier selon l'une quelconque des revendications 1 à 9, dans lequel le matériau à changement de phase (115) est combiné à un matériau conducteur de chaleur divisé.

11. Boîtier selon l'une quelconque des revendications 1 à 10, comprenant, en outre, au moins un panneau de cellules photovoltaïques (141) relié à une batterie (140) elle-même reliée à un microcontrôleur (150) apte à commander le fonctionnement d'au moins un équipement électrique incorporé au boîtier.

12. Boîtier selon la revendication 11, dans lequel le microcontrôleur (150) est apte à commander le fonctionnement d'une résistance électrique (117) en contact thermique avec le volume de matériau à changement de phase (115).

13. Boîtier selon l'une quelconque des revendications 11 ou 12, dans lequel le microcontrôleur (150) est relié à un émetteur/récepteur (151) de liaison sans fil à faible consommation énergétique, capable d'émettre et de recevoir des données sur un réseau étendu sans fil à faible consommation énergétique, dit « LPWAN » et/ou sur un réseau local sans fil à faible consommation énergétique, dit « LPLAN ».

14. Boîtier selon l'une quelconque des revendications 1 1 à 13, comprenant, en outre, au moins un ventilateur (130) commandé par le microcontrôleur (150).

15. Boîtier selon l'une quelconque des revendications 11 à 14, comprenant, en outre, au moins un équipement électronique de contrôle (160) relié au microcontrôleur (150).

16. Boîtier selon la revendication 15, dans lequel l'équipement électronique de contrôle (160) est choisi parmi une sonde de température, un capteur d'hygrométrie, un capteur d'état de l'objet stocké, un contact sec, ou un assortiment de ceux-ci.

17. Boîtier selon l'une quelconque des revendications 1 à 16, dans lequel les orifices de passage d'air (112a-112b) sont équipés chacun d'une trappe commandable (113a-113b) entre une position ouverte autorisant un passage d'air et une position fermée bloquant le passage de l'air.

18. Boîtier selon l'une quelconque des revendications 1 à 17, comprenant, en outre, des orifices d'entrée/sortie d'air (230, 231, 232) commandables entre une position ouverte autorisant un passage d'air de ou vers l'extérieur du boîtier et une position fermée bloquant le passage de l'air.

19. Boîtier selon la revendication 18, dans lequel la chambre de stockage (201) comprend deux orifices d'entrée/sortie d'air (230, 231), et la chambre de régulation (210) comprend un orifice d'entrée/sortie d'air (232).

## Patentansprüche

1. Autonomes, wärmereguliertes Gehäuse (100, 200, 300, 400), umfassend:
• eine Speicherkammer (101, 201, 301, 401), die mit einer Schicht aus wärmedämmendem Material (102) abgedeckt und mit einem Träger für ein zu speicherndes Produkt (P) versehen ist;
• eine Regulierungskammer (110, 210, 310, 410), die von der Speicherkammer (101, 201, 301, 401) durch eine Trennwand (111) getrennt ist, die mit zwei Luft-Durchgangsöffnungen (112a - 112b, 213a - 213b) versehen ist, und umfassend
- eine Kanalisationswand (114), die zwischen den zwei Luft-Durchgangsöffnungen (112a - 112b, 213a - 213b) befestigt ist, die einen Luft-Umlaufkreislauf mit zwei Luft-Umlaufsäulen und einem Durchgang zwischen diesen zwei Säulen begrenzt;
- ein bestimmtes Volumen eines Materials mit Phasenänderung (115);
**gekennzeichnet durch**
• ein Heizmittel (116, 117, 118 - 119, 221) des Materials mit Phasenänderung (115).

2. Gehäuse gemäß Anspruch 1, bei dem die Regulierungskammer (110) unter Bezugnahme auf die Nutzungsposition unter der Speicherkammer (101) angeordnet ist.

3. Gehäuse gemäß Anspruch 1, bei dem die Regulierungskammer (310) unter Bezugnahme auf die Nutzungsposition in Bezug auf die Speicherkammer (301) seitlich angeordnet ist.

4. Gehäuse gemäß Anspruch 1, bei dem die Regulierungskammer (410) unter Bezugnahme auf die Nutzungsposition oberhalb der Speicherkammer (401) angeordnet ist.

5. Gehäuse gemäß Anspruch 1, bei dem die Regulierungskammer (110) durch eine wärmeleitende Wand (116) begrenzt ist.

6. Gehäuse gemäß irgendeinem der Ansprüche 1 bis 5, bei dem die Regulierungskammer (110, 210) mit einer Verglasung (118) auf einer Seite versehen ist, die dazu bestimmt ist, in der Nutzungsposition der Sonne ausgesetzt zu sein und bei der das Materialvolumen mit Phasenänderung (115) mit einer Wärmeauffangplatte (119, 221) abgedeckt ist, die gegenüber der Verglasung (118) angeordnet ist, wobei die Verglasung und die Wärmeauffangplatte ein Heizmittel des Materials mit Phasenänderung bildet.

7. Gehäuse gemäß irgendeinem der Ansprüche 1 bis 6, bei dem das Materialvolumen mit Phasenänderung (115) von der Kanalisationswand (114) gestützt ist.

8. Gehäuse gemäß irgendeinem der Ansprüche 1 bis 6, bei dem das Materialvolumen mit Phasenänderung (115) von einer Außenwand (116, 216) der unteren Regulierungskammer (110) gestützt ist.

9. Gehäuse gemäß irgendeinem der Ansprüche 7 oder 8, bei dem das bestimmte Materialvolumen mit Phasenänderung (115) entfernt von der Wand (114, 116, 216), die sie trägt, derart befestigt ist, dass ein doppelter Luftumlaufkreislauf auf jeder Seite des Materialvolumens mit Phasenänderung eingerichtet ist.

10. Gehäuse gemäß irgendeinem der Ansprüche 1 bis 9, bei dem das Material mit Phasenänderung (115) mit einem geteilten, wärmeleitenden Material kombiniert ist.

11. Gehäuse gemäß irgendeinem der Ansprüche 1 bis 10, umfassend darüber hinaus wenigstens ein photovoltaisches Zellenpaneel (141), das mit einer Batterie (140) verbunden ist, die ihrerseits mit einem Microcontroller (150) verbunden ist, der geeignet ist, die Funktionsweise wenigstens einer in das Gehäuse eingebauten elektrischen Ausrüstung zu steuern.

12. Gehäuse gemäß Anspruch 11, bei dem der Microcontroller (150) geeignet ist, die Funktionsweise eines elektrischen Widerstandes (117), der in thermischem Kontakt mit dem Materialvolumen mit Phasenänderung (115) ist, zu steuern.

13. Gehäuse gemäß irgendeinem der Ansprüche 11 oder 12, bei dem der Microcontroller (150) mit einem Sender / Empfänger (151) mit drahtloser Verbindung mit geringem Energieverbrauch verbunden ist, der geeignet ist, Daten auf einem weiten Netzwerk mit geringem Energieverbrauch, bezeichnet als "LPWAND" und / oder in einem drahtlosen lokalen Netzwerk mit geringem Energieverbrauch, bezeichnet als "LPAN", zu senden und zu empfangen.

14. Gehäuse gemäß irgendeinem der Ansprüche 11 bis 13, umfassend darüber hinaus wenigstens einen Belüfter (130), der vom Microcontroller (150) gesteuert ist.

15. Gehäuse gemäß irgendeinem der Ansprüche 11 bis 14, umfassend darüber hinaus wenigstens eine elektronische Kontrollausrüstung (160), die mit dem Microcontroller (150) verbunden ist.

16. Gehäuse gemäß Anspruch 15, bei dem die elektronische Kontrollausrüstung (160) aus einer Temperatursonde, einem Luftfeuchtigkeitssensor, einem Zustandssensor des gespeicherten Objekts, einem trockenen Kontakt oder einer Zusammenstellung derselben ausgewählt ist.

17. Gehäuse gemäß irgendeinem der Ansprüche 1 bis 16, bei dem die Luft-Durchgangsöffnungen (112a - 112b) jeweils mit einer Klappe (113a - 113b) ausgerüstet sind, die zwischen einer offenen Position, die einen Luftdurchgang zulässt, und einer geschlossenen Position, die den Luftdurchgang blockiert, steuerbar ist.

18. Gehäuse gemäß irgendeinem der Ansprüche 1 bis 17, umfassend darüber hinaus Lufteingangs- / ausgansöffnungen (230, 231, 232), die zwischen einer offenen Position, die einen Luftdurchlass von oder zur Außenseite des Gehäuses zulässt, und einer geschlossenen Position, die den Durchlass der Luft blockiert, steuerbar ist.

19. Gehäuse gemäß Anspruch 18, bei dem die Speicherkammer (201) zwei Lufteingangs- / -ausgangsöffnungen (230, 231) umfasst und die Regulierungskammer (210) eine Lufteingangs- / -ausgangsöffnung (232) umfasst.

## Claims

1. A stand-alone thermo regulated casing (100, 200, 300, 400) comprising:
• a storage chamber (101, 201, 301, 401), covered with a thermally insulating material layer (102), and provided with a support for a product (P) to be stored;
• a regulation chamber (110, 210, 310, 410), separated from the storage chamber (101, 201, 301, 401) by a partition wall (111) provided with two air ports (112a-112b, 213a-213b), and comprising
- a piping wall (114) attached between both air ports (112a-112b, 213a-213b), delimiting an air circulation circuit with two air circulation columns and a passage between these two columns;
- a determined volume of a phase change material (115);
**characterised by**
• a heating means (116, 117, 118-119, 221) for the phase change material (115).

2. The casing according to claim 1, wherein, with reference to the position of use, the regulation chamber (110) is arranged under the storage chamber (101).

3. The casing according to claim 1, wherein, with reference to the position of use, the regulation chamber (310) is arranged laterally relative to the storage chamber (301).

4. The casing according to claim 1, wherein, with reference to the position of use, the regulation chamber (410) is arranged above the storage chamber (401).

5. The casing according to claim 1, wherein the regulation chamber (110) is delimited by a thermally conducting wall (116).

6. The casing according to any of claims 1 to 5, wherein the regulation chamber (110, 210) is provided with a glazing (118) on a face to be exposed to the sun, in a position of use, and wherein the volume of phase change material (115) is covered with a heat collecting plate (119, 221) arranged facing the glazing (118), the glazing and heat collecting plate making up a means for heating the phase change material.

7. The casing according to any of claims 1 to 6, wherein the volume of phase change material (115) is supported by the piping wall (114).

8. The casing according to any of claims 1 to 6, wherein the volume of phase change material (115) is supported by an external wall (116, 216) of the lower regulation chamber (110).

9. The casing according to any of claims 7 or 8, wherein the determined volume of phase change material (115) is attached at a distance from the wall (114, 116, 216) carrying it, so as to provide a double air circulation circuit on either side of the volume of phase change material.

10. The casing according to any of claims 1 to 9, wherein the phase change material (115) is combined with a divided heat conducting material.

11. The casing according to any of claims 1 to 10, further comprising at least one photovoltaic cell panel (141) connected to a battery (140) in turn connected to a microcontroller (150) able to control the operation of at least one piece of electric equipment embedded into the casing.

12. The casing according to claim 11, wherein the microcontroller (150) is able to control the operation of an electric resistor (117) in thermal contact with the volume of phase change material (115).

13. The casing according to any of claims 11 or 12, wherein the microcontroller (150) is connected to a low power consumption wireless link transceiver (151), able to transmit and receive data over a so-called wireless low power wide area network, "LPWAN" and/or over a so-called wireless low power local area network, "LPLAN".

14. The casing according to any of claims 11 to 13, further comprising at least one fan (130) controlled by the microcontroller (150).

15. The casing according to any of claims 11 to 14, further comprising at least one piece of electronic control equipment (160) connected to the microcontroller (150).

16. The casing according to claim 15, wherein the electronic control equipment (160) is selected from a temperature probe, a hygrometry sensor, a condition sensor for the stored object, a dry contact, or a combination thereof.

17. The casing according to any of claims 1 to 16, wherein the air ports (112a-112b) are each fitted with a trap door (113a-113b) controllable between an open position allowing air passage and a closed position blocking air passage.

18. The casing according to any of claims 1 to 17, further comprising, air inlet/outlet ports (230, 231, 232) controllable between an open position allowing air passage from or to outside of the casing and a closed position blocking air passage.

19. The casing according to claim 18, wherein the storage chamber (201) comprises two air inlet/outlet ports (230, 231), and the regulation chamber (210) comprises one air inlet/outlet port (232).
